# EUROPEAN PATENT APPLICATION

(11) **EP 2 796 873 A1**
(43) Date of publication of application: **29.10.2014**
(21) Application number: 13165419.6
(22) Date of filing: 25.04.2013
(51) Int. Cl.: G01N 33/50

(54) **Method for a cell-based drug screening assay and the use thereof**

(71) Applicant: QGel SA, 1015 Lausanne (CH)
(72) Inventor: Rizzi, Simone, 6883 Novazzano (CH); Jacot, Guillaume, 1004 Lausanne (CH); Lütolf, Matthias, 1131 Tolochenaz (CH)
(74) Representative: Hepp Wenger Ryffel AG

(57) **Abstract**

The present invention relates to a cell-based drug screening assay comprising the steps of:
(i) culturing a cell population in a culturing environment,
(ii) defining at least two monitoring points in time in dependency of at least one of the cell types, physiological characteristics of the cells, physiological characteristics of formed-tissue, the mode of action of a drug substance and the culturing environment, (iii) applying drug substances to the cultured cells at least at one treatment point in time,
(iv) monitoring of the effect of said drug substance on cells or formed tissues at least at said two monitoring points in time.

## Description

The present invention relates to a drug screening method on cells cultured in a culturing environment according to the preamble of the independent claim 1.

Cell-based screening methods represent a crucial source of information in the decision-making process to evaluate mode of action, such as efficacy and toxicity, of new compounds, e.g. new anti-cancer drugs, in early phases of preclinical drug development (Michelini, Cevenini et al., (2010) "Cell-based assays: fuelling drug discovery." Anal Bioanal Chem 398(1): 227-238.; An and Tolliday (2010), "Cell-based assays for high-throughput screening." Mol Biotechnol 45(2): 180-186; Sharma, Haber et al., (2010) "Cell line-based platforms to evaluate the therapeutic efficacy of candidate anticancer agents." Nature Reviews Cancer 10: 241-253.).

Given their key role in drug development, a large number of novel approaches were investigated in the last decade towards the development of more physiologically relevant cell-based assays (Yamada and Cukierman (2007), "Modeling tissue morphogenesis and cancer in 3D." Cell 130(4): 601-610; Pampaloni, Reynaud et al. (2007); "The third dimension bridges the gap between cell culture and live tissue." Nat Rev Mol Cell Biol 8(10): 839-845). For instance, in cancer cell biology and anti-tumour drug development, a growing body of scientific evidence suggests that methods allowing cancer cells to organize and grow in three-dimensional structures (three-dimensional culture models), compared to conventional cultures on flat plastic surfaces (two-dimensional culture models), reflect more closely behaviours observed in vivo; in particular, with regard to cell growth, cell signalling and gene expression (Abbott (2003). "Cell culture: Biology's new dimension." Nature 424(6951): 870-872, Kunz-Schughart, Freyer et al. (2004), "The use of 3-D cultures for high-throughput screening: the multicellular spheroid model." J Biomol Screen 9(4): 273-285; Bissell (2007), "Modelling molecular mechanisms of breast cancer and invasion: lessons from the normal gland." Biochem Soc Trans 35(Pt 1): 18-22.; Friedrich, Seidel et al. (2009). "Spheroid-based drug screen: considerations and practical approach." Nat Protoc 4(3): 309-324; Debnath and Brugge (2005), "Modelling glandular epithelial cancers in three-dimensional cultures." Nat Rev Cancer 5(9): 675-688).

Several techniques have been employed to produce these more physiological three-dimensional culture models to test anticancer drugs (Nyga 2011, Cheema et al.(2011), "3D tumour models: novel in vitro approaches to cancer studies" J. Cell Commun. Signal. 5, 239-248). The most common three-dimensional techniques use either (i) gel matrices from natural (Weaver, Petersen et al. (1997), "Reversion of the malignant phenotype of human breast cells in three-dimensional culture and in vivo by integrin blocking antibodies." J Cell Biol 137(1): 231-245.; Fiebig, Maier et al. (2004), "Clonogenic assay with established human tumour xenografts: correlation of in vitro to in vivo activity as a basis for anticancer drug discovery." Eur J Cancer 40(6): 802-820; Krausz, de Hoogt et al. (2013), "Translation of a tumor microenvironment mimicking 3D tumor growth co-culture assay platform to high-content screening." J Biomol Screen 18(1): 54-66) or synthetic (Loessner, Stok et al. (2010). "Bioengineered 3D platform to explore cell-ECM interactions and drug resistance of epithelial ovarian cancer cells." Biomaterials 31(32): 8494-8506; Sieh, Taubenberger et al. (2012), "Phenotypic characterization of prostate cancer LNCaP cells cultured within a bioengineered microenvironment." PLoS One 7(9): e40217.; Yang and Zhao (2011). "A 3D model of ovarian cancer cell lines on peptide nanofiber scaffold to explore the cell-scaffold interaction and chemotherapeutic resistance of anticancer drugs." Int J Nanomedicine 6: 303-310) origin for cell encapsulation, (ii) rigid polymeric three-dimensional scaffolds (Fischbach, Chen et al. (2007), "Engineering tumors with 3D scaffolds." Nat Methods 4 (10) : 855-860; Knight, Murray et al. (2011). "Alvetex(R) : polystyrene scaffold technology for routine three dimensional cell culture." Methods Mol Biol 695: 323-340) for cell seeding, or (iii) matrix-free cell aggregation processes (WO 2010/031194 A1;(Friedrich, Seidel et al. (2009), "Spheroid-based drug screen: considerations and practical approach." Nat Protoc 4(3): 309-324; Karlsson, Fryknas et al. (2012). "Loss of cancer drug activity in colon cancer HCT-116 cells during spheroid formation in a new 3-D spheroid cell culture system." Exp Cell Res 318(13): 1577-1585; Tung, Hsiao et al. (2011). "High-throughput 3D spheroid culture and drug testing using a 384 hanging drop array." Analyst 136(3): 473-478; Lama, Zhang et al. (2013). "Development, validation and pilot screening of an in vitro multicellular three-dimensional cancer spheroid assay for anti-cancer drug testing." Bioorg Med Chem 21(4): 922-931) to grow three-dimensional cancer cell structures (e.g. spheroids, tissue).

The failure to take into account the variation in disease progression may increase the probability to select false-positive drug candidates (drugs that will not work in humans) and to discard false-negative drug candidates (drugs that would have worked in human).

It is therefore an objective of the present invention to avoid disadvantages of the known drug screening methods, and specifically to provide data on drug activities tested on profiled cells displaying different key physiological in vivo disease states. This objective is solved with the drug screening method according to claim 1.

According to the present innovation the cell-based drug screening assay takes into account cells as well as their physiological changes and behaviour over time to understand the mode of action of drugs. It comprises determining at least two monitoring points in time in dependency of the cell type, the culturing environment, the physiological characteristics of the cells, the physiological characteristics of cell populations, the physiological characteristics of formed tissue, or the kind of drug substance, e.g. the targeted mechanism of action of a drug substance. The drug substances are applied to the cultured cells at least at one treatment point in time. The effect of a drug type on cell growth kinetics, behaviour and/or physiological characteristics are monitored at least at said two monitoring points in time. The points in time can be a specific moment but also a certain period of time.

The culturing environment includes physiological relevant microenvironments which take into account the specific physical, biological and biochemical features including cell-cell and cell-matrix interactions for specific cells and/or formed tissues.

Monitoring of cell proliferation at least at two points in time, for example at an "early-stage" and at a "late-stage", may allow comparison of two stages during cell growth and/or development of physiological features and/or effect of drug treatment. The cell-growth environment and physiological features may need to be adjusted for a given cell-type or drugs. The point in time of the monitoring is thus preferably chosen such as to provide information relevant in view of mode of action of a drug to e.g. early or late stages of cancer; a specific cancer may be considered as "early stage" preferably four to seven days after culturing in a physiologically relevant environment, and "late-stage" preferably fourteen to seventeen days after culturing in a physiologically relevant environment. "Late-stage" may be also defined by the absence or presence of a specific physiological characteristic of feature, e.g. hypoxia or changes in cell growth kinetics or constitution of cells.

Monitoring of cells may continue one to seven or more days after termination of the drug application. The start and end of the drug-free time period has to be determined for a specific cell type and drug. As the cells are allowed to recover from the drug treatment this period is named "recovery phase". Including points in time relevant for a recovery after a first and/or a second drug application may be essential for the identification of effective drugs or drugs that do not have an immediate effect on cells.

Preferred embodiments may refer e.g. to a process including treatment at an "early-stage" and monitoring at least at two related points in time with or without a "recovery phase", or treatment at a "late-stage" and monitoring at least at two related points in time with or without a "recovery phase", or treatments at an "early-stage" and a "late-stage" and monitoring at least two related points in time with or without a "recovery phase".

The environment used for cell culturing may comprise a substrate matrix or medium that mimics the cellular microenvironment, preferably a three-dimensional matrix that consists of a structural compound and a linker compound to facilitate polymerization. The structural compound may be multi-branched polyethylene glycol comprising unsaturated end groups, preferably vinyl groups. The linker compound comprises a peptide with at least two cysteines or thiol groups enabling polymerization of the structural compound bridged by means of the linker compound. The three-dimensional matrix preferably comprises a matrix composition as described in the European Publication Number 2 561 005.

The environment used for cell culturing may promote three-dimensional growth (i) of a single cell to form a multicellular entity or (ii) of multiple cells to form a multicellular entity, when they are seeded into said environment. Preferentially, the cells grow to form a cell colony or a cell population. For example, colony arrangements of cancer cells form spheroids that mimic the three-dimensional arrangement of tumours where outer cells are easily accessible and at the same time shield cells at its core.

In order to produce a three-dimensional environment with a defined stiffness and a composition with appropriate physiological and biological characteristics, a recipient or a standard cell-culture device may be used, preferably a 384-well plate. The said environment and the cells may be dispensed into the device with cell culture media which may then be placed in a cell culture incubator. Monitoring of cell proliferation and activity may be measured using imaging and sensory equipment that is compatible with the recipient or the device, preferably equipment that is automated and maximize the reproducibility of the output monitoring data.

The cells used for culturing in the said environment may be any type of cell of any organism, cell or cell line cultured in vitro, differentiated or stem cell derived from an organism and cancer cell of an organism, preferentially colon, pancreatic and ovarian cancer cell, but also healthy cells of which the growth is promoted.

The drug substances applied to cultured cells may be any substances selected of the group of any chemical, natural or synthetic substance, any substance of known drug screens and substances recognized before as anti-cancer drugs. Drug substances may be applied in liquid form, wherein the liquid may be an organic solvent or an aqueous buffer comprising the drug substance, as powder, as pill or encapsulated in a carrier, preferably a nanoparticle.

Assay readout modality to monitor cell growth kinetics and activity may be carried out by preferably any (i) of the selected group of metabolic activity, preferably Alamar Blue@, flow cytometry, Deoxyribonucleic acid (DNA) content, protein quantification, cell counting, image-based analysis, gene expression levels, mass spectrometry, label-based measurements (such as reporter gene technology, resonance energy transfer, protein-fragment- or split- complementation assays), or label-free based measurements (such as electrical biosensor/impedance systems, optical biosensor systems, surface plasmon resonance systems, acoustic biosensor systems, and systems that exploit the calorimetry, change in electrical activity or by (ii) any combination of afore mentioned monitoring assays. Monitoring of cell proliferation gives direct information on the drug-induced effect on cellular shape and constitution.

Assay readout modality to determine other physiological and biological characteristics (e.g. hypoxic and/or angiogenic markers) may be carried out preferably by any (i) of the selected group of Ribonucleic acid (RNA) levels, protein expression levels, mass spectrometry, immunohistochemistry, image-based analysis, proteomics methods or by (ii) any combination of aforementioned monitoring assays.

The maximally tolerated dose or concentration of an applied drug substance may be determined for a given cell and cell type. Determination of the accepted drug dose of a cell, cell type or tissue allows adjustment of the applied drug doses when targeting diseased cells, preferably cancer cells.

The lowest effective dose of an applied drug substance may be determined for a given cell and cell type. Determination of the lowest effective dose of a cell, cell type or tissue allows adjustment of the applied drug doses, when targeting diseased cells, preferably cancer cells.

Another objective of the present invention is to provide use of the method to monitor (i) curing or killing diseased cells, supporting healthy cells and inducing healthy cells to enter a state of disease by drug treatment or (ii) reprogramming and/or differentiation of cells.

Reprogramming of differentiated cells into stem cells or progenitor cells with respect to an applied drug substance may be assayed by any (i) of the selected group of metabolic activity, preferably Alamar Blue@, flow cytometry, Deoxyribonucleic acid (DNA) content, protein quantification, cell counting, image-based analysis, gene expression levels, mass spectrometry, label-based measurements (such as reporter gene technology, resonance energy transfer, protein-fragment- or split- complementation assays), or label-free based measurements (such as electrical biosensor/impedance systems, optical biosensor systems, surface plasmon resonance systems, acoustic biosensor systems, and systems that exploit the calorimetry, change in electrical activity or by (ii) any combination of afore mentioned monitoring assays. The differentiated cell may be any cell, cell type, cell culture-derived cell or cell derived from a specific tissue. Here, the applied drug substance may trigger the reprogramming process.

The method may be used for analyzing the differentiation of stem cells into a differentiated cell with respect to an applied drug substance according to cell cell growth kinetics and activity including any (i) of the selected group of metabolic activity using Alamar Blue®, flow cytometrie, hypoxic and angiogenic markers, Ribonucleic acid (RNA) levels, protein expression levels, mass spectrometry, DNA content, protein quantification, cell counting, image-based analysis, and immunohistochemistry or by (ii) any combination of afore mentioned monitoring assays. Here, the applied drug substance may trigger the differentiation process.

Further aspects and details of the present invention will become apparent from the figures and examples given in the following, which show:
Fig. 1: Graphs and images showing cell colony profiling and hypoxic conditions of Colon cancer cells (HCT 116) in a three-dimensional matrix;
Fig. 2: Schematic representation showing the determination of a drug treatment schedule in a three-dimensional physiological microenvironment as well as for conventional two-dimensional monolayer culture;
Fig. 3: Graph comparing two-dimensional vs. three-dimensional screening of drugs using the determined drug treatment schedule in Fig 2;
Fig. 4: Graphs and images showing the determination of dose-dependant drug efficacy profiles after "early-" and "late-stage" treatment;
Fig. 5: Graph comparing efficacy of drug activities measured immediately after treatment end and after a drug-free period;
Fig. 6: Table to summarize the 5-Fluorouracil drug efficacy against colony of HCT 116 cells grown in a three-dimensional environment and in conventional 2D cell culture models;
Fig. 7: Graph showing the two-dimensional growth of Colon cancer cells (HCT 116);
Fig. 8: Graphs and images showing a comparison of respective growth in three-dimensional environments of Colon, Pancreatic and Ovarian cancer cells;
Fig. 9: Graphs showing growth profiles for two-dimensional and three-dimensional cell cultures with independent treatment schedules;
Fig. 10: Graph showing pancreatic cancer cells treated with Gemcitabine: Comparison of measuring drug activity immediately after treatment end and after four and seven days of a drug-free recovery period.

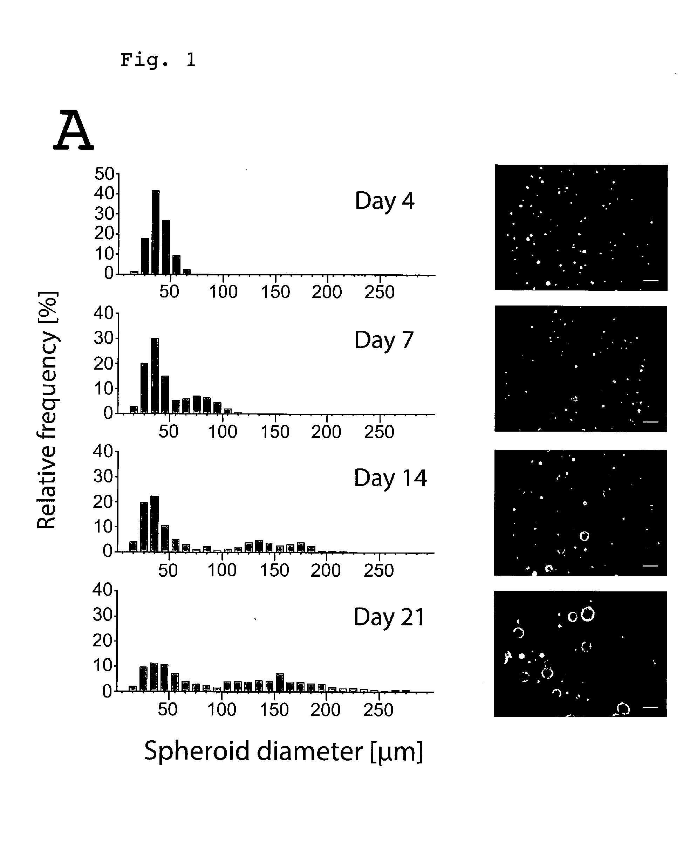

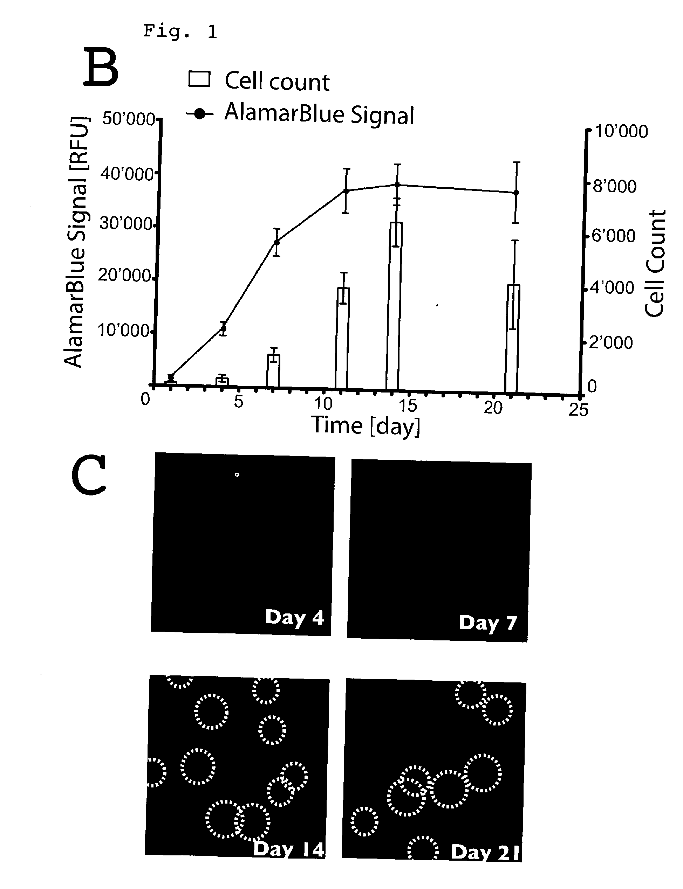

Figure 1 shows growth profile of Colon cancer cells (HCT 116) in a three-dimensional physiological microenvironment without drug treatment. In A) cells grow as a colony of cells or cancer spheroids mimicking mini-tumours, when encapsulated as single cells. The spheroid size increases over time. The left panel shows image-based analysis represented as histograms quantifying the distribution of spheroid sizes formed at different time points. Spheroid diameter size of cancer cells increases from 35 µm at day four to 155 µm at day twenty-one of growth. The right panel shows fluorescent calcein-based live staining images of growing cancer cells. Growth kinetics of cancer cell spheroids in a three-dimensional matrix is shown in B). Metabolic activity by Alamar Blue@ and cell counting by flow cytometrie are depicted, both performed at optimized assay conditions showing similar cell growth profiles characterized by initially fast and subsequently flattening cell growth kinetics. In C) cell spheroids were stained with calceine (green) for live cells and with hypoxisense (red) for cells in hypoxic conditions. In this cancer cell line, hypoxia identified in red (normally in the centre of the cell spheroids) was observed from around day 14 in culture. For visualization in grey scale spheroids exhibiting red staining are indicated with dotted circles. The red staining is most intense at the centre of the spheroid.

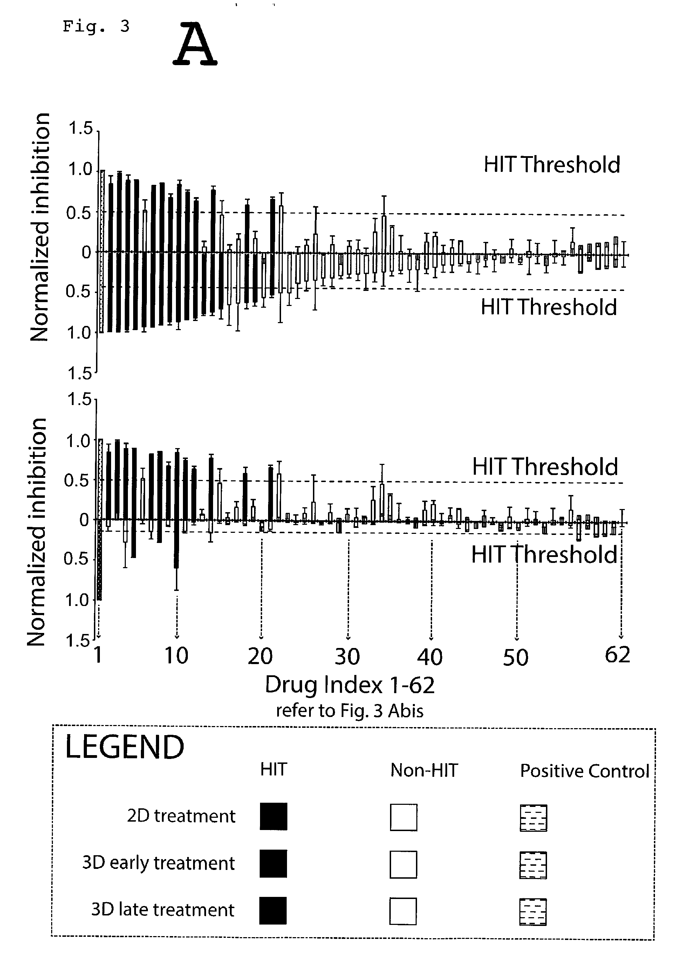

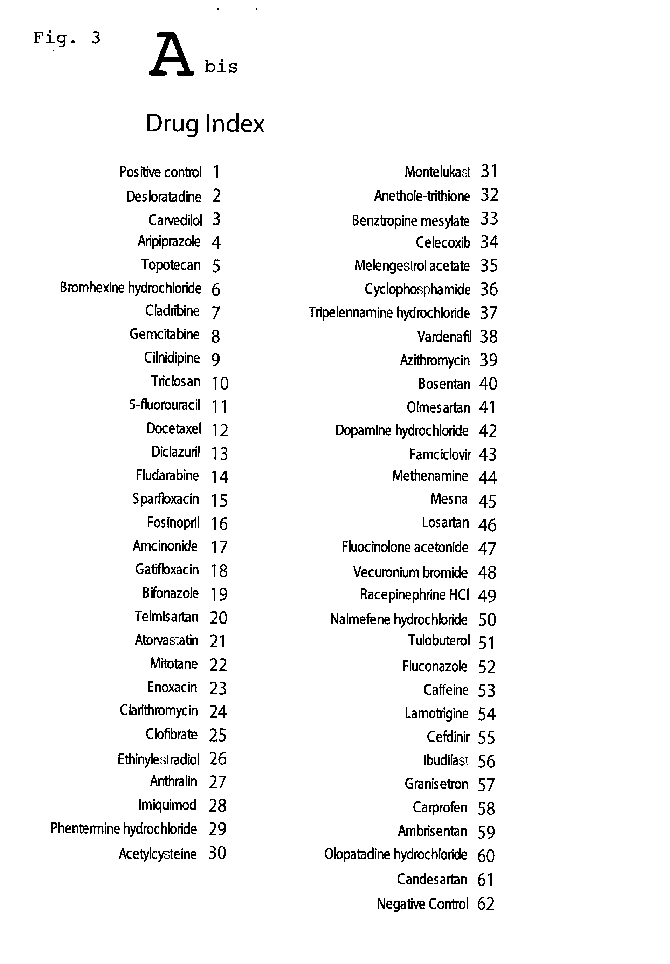

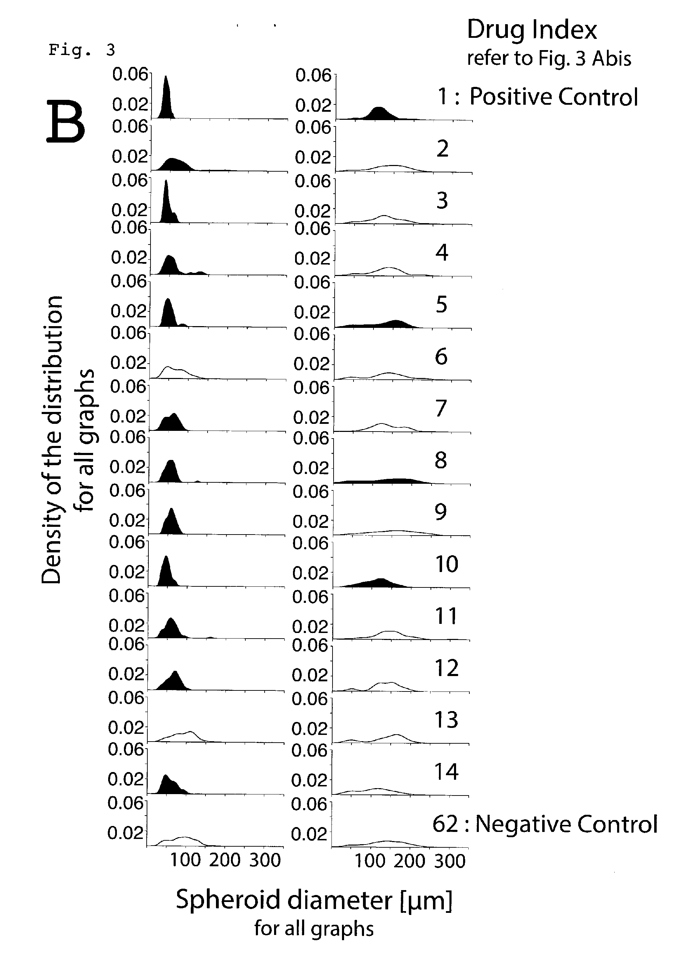

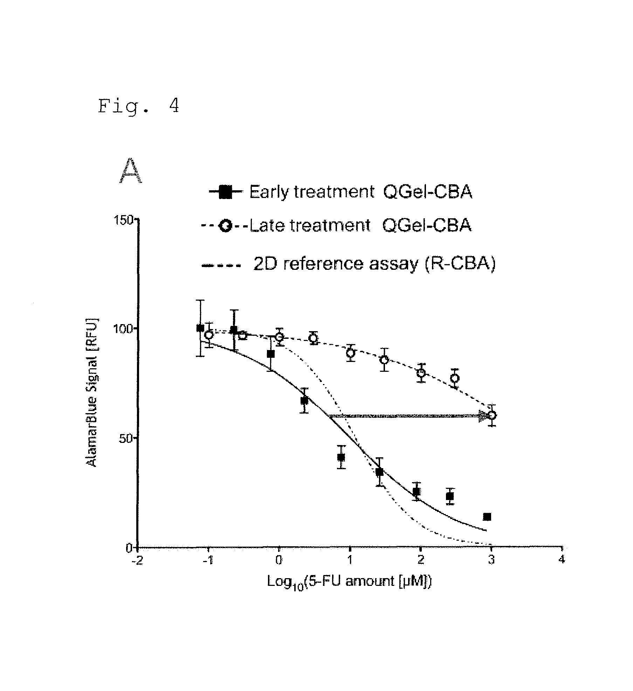

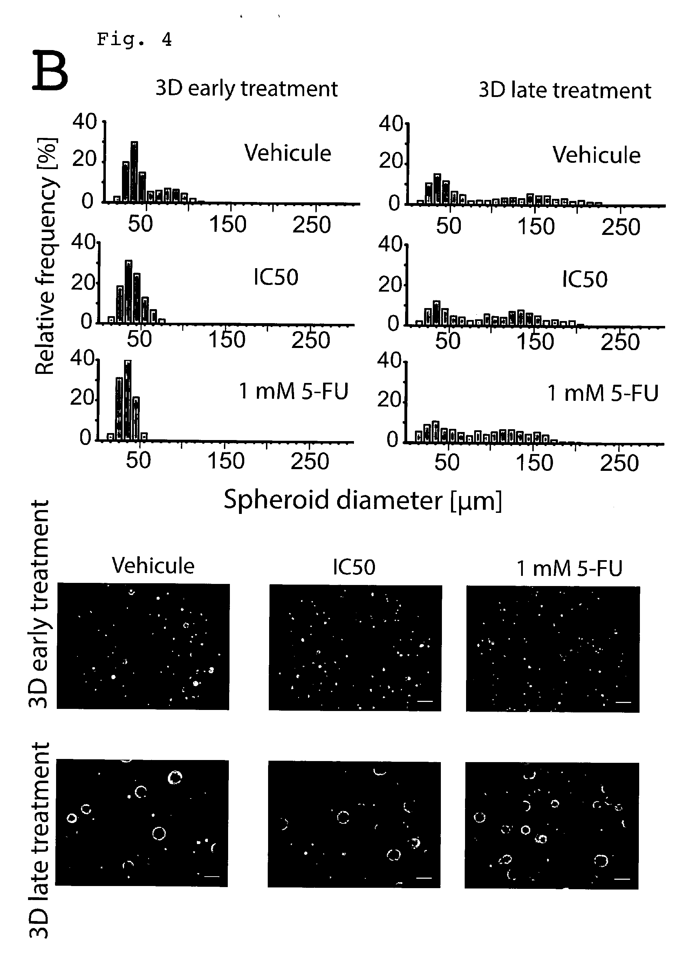

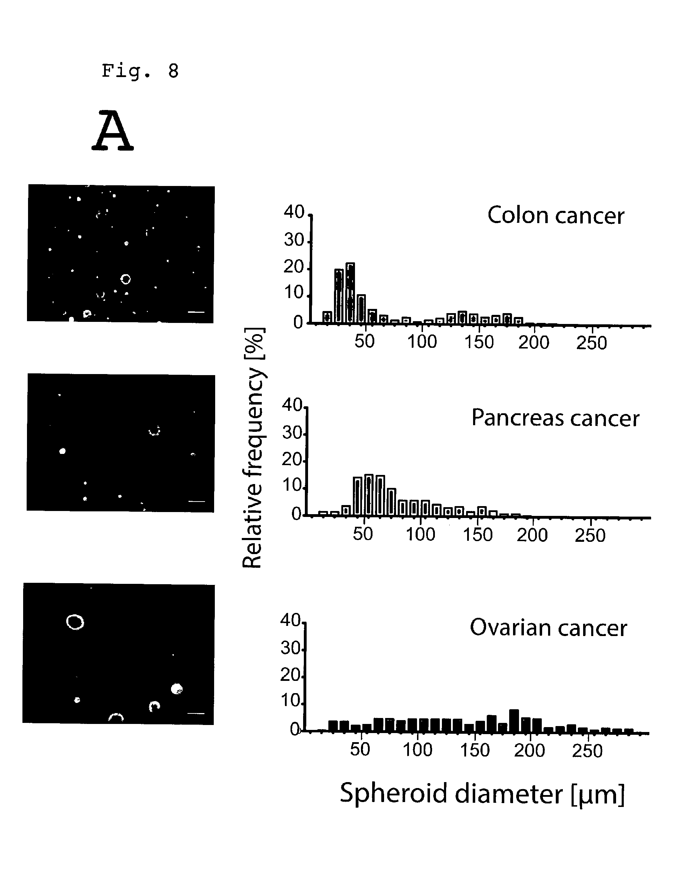

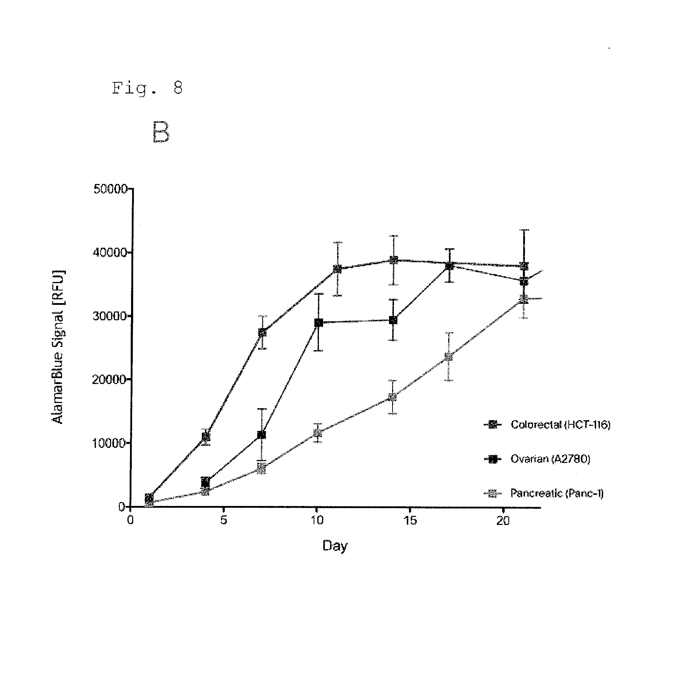

Profiling of cancer cells is a key step in the present invention, as it permits to identify and investigate physiological characteristics (e.g. hypoxia) of cancer cell spheroids at different time points of growth. Importantly, these characteristics strongly depend on the origin of tumour tissue (as shown in figure 8). In addition, drug efficacy on tumours strongly depends on features of tumour physiology (as shown in figures 3 and 4). Based on profiled physiological characteristics of cancer cell spheroids in the present invention, treatment time points for drug screening may be adapted thereafter to mimic different stages of tumours in vivo. Spheroids in the gel matrix displaying different stages of cell proliferation are demonstrated in figure 1 A and 8B.

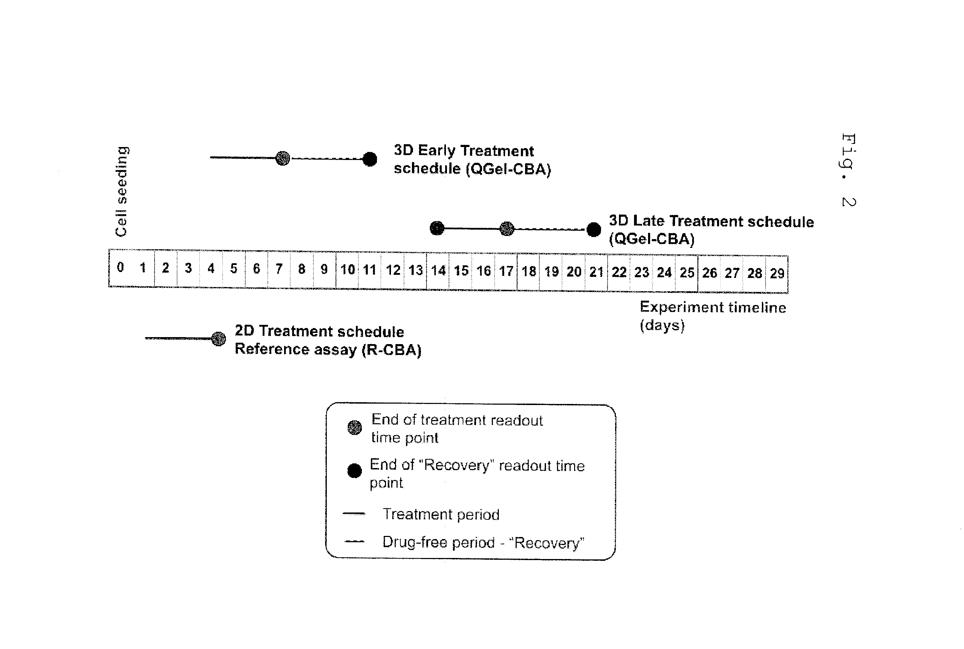

Figure 2 shows the determination of a drug treatment schedule and readout time points based on profiled cell growth and physiological characteristics of cancer. An example is given comprising an "early-" and a "late-stage" drug treatment for cells grown in three-dimensional matrices. Conditions of different cancer stages are identified, derived from profiling assays, as in Figure 1, and schedules for drug screening are determined accordingly. Here, different cancer stages for cells cultured in three-dimensional matrices are determined as "early-" and "late-stage" cancer treatment.

The "early-stage" comprises two time points for readout. The first point of time at day seven is at the end of the drug treatment, whereas the second point in time at day eleven follows a drug-free recovery phase. The "late-stage" comprises two time points for readout. The first point in time at day seventeen is at the end of a second (independent from "early-stage") drug treatment phase. The second point in time at day twenty-one follows a drug-free recovery phase. Measurements at additional points in time can be performed before the start of treatment and/or after different drug-free recovery phases.

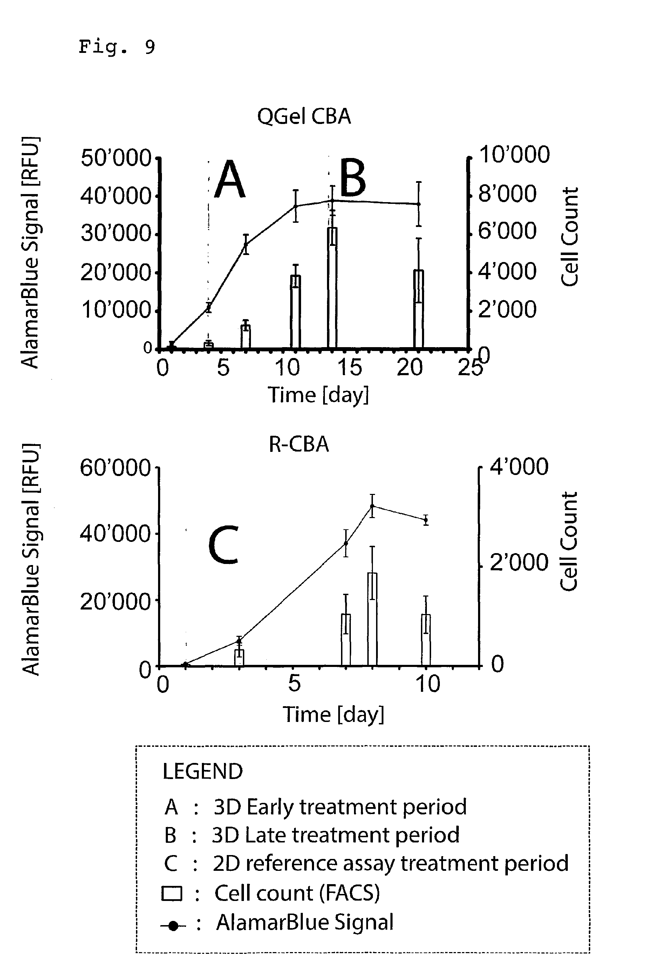

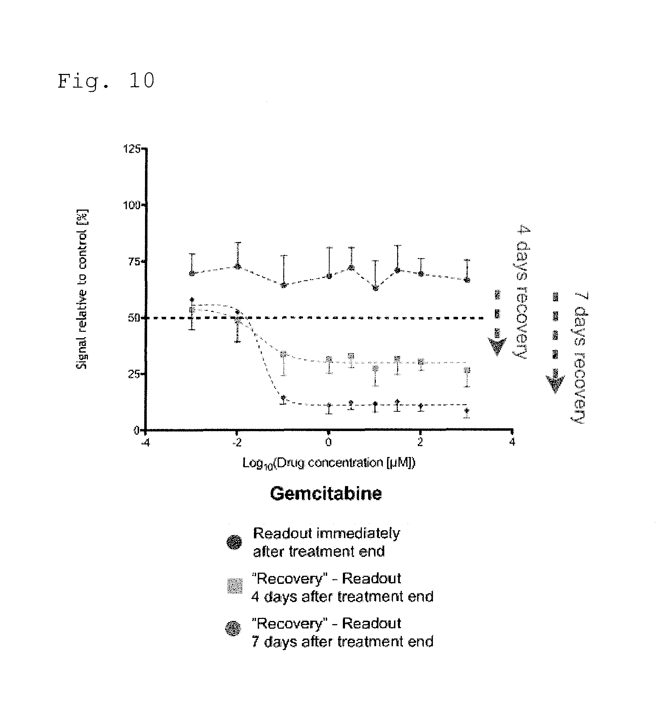

The "early-stage", day four to seven, shows small spheroids with a diameter of 35 µm, highly proliferative cells (shown in figure 9) and no hypoxic conditions determined with measurements of hypoxic marker expression or other biochemical means (figure 1C). In contrary, the "late-stage" illustrates existence of larger spheroids with a diameter of 155 µm, low cell proliferation (shown in fig. 9), and presence of hypoxic condition (figure 1C). In both cases, the readouts are performed immediately after treatment end to test immediate drug efficacy, and after a four-day drug-free period to test recurrence of "mini-tumour" growth and/or retarded drug effects as the case observed with Gemcitabine (Figure 10).

For reference two-dimensional assays, cells are normally tested with drugs within the logarithmic proliferations phase between day one to four (figure 9).

Figure 3 shows a drug screening campaign using a treatment schedule as described in figure 2 on colonies of Colon cancer HCT 116 cells. As an example for "Hit" generation a selection of drugs from a commercially available drug library (Prestwick) is used. In A) the effect of sixty drugs on cancer cells measured by metabolic readout (AlamarBlue®) is shown. The compounds are tested in triplicate at concentration of 10 µM. The means and standard deviations are shown here. A drug is considered as "Hit" only if its three replicates are above the HIT threshold (mean +/- three times the standard deviations of the negative control. Conditions with DMSO vehicle lacking the drug are used as negative controls). The upper chart shows a head to head comparison of early treatment of cancer cells grown in two-dimensions and embedded in a three-dimensional matrix, the lower chart comparison of "early-" and "late-stage" treatment of embedded cells in a three-dimensional matrix. It displays that with the "early-" and "late-stage" treatment identification of false-positive drugs is efficiently avoided reducing the number of hits.

In figure 3 B an image-based analysis quantifying the distribution of diameter sizes of cell colonies, specifically cancer spheroids measured immediately after "early-" and "late-stage" treatment is shown as an example of a selection of drugs from figure 3 A at a drug concentration of 10 µM. Filled curves correspond to "Hits" and unfilled curves are "no Hits" as determined using metabolic readouts at the ends of "early stage" and "late-stage" (Positive control: Cells treated with copper sulphate; negative control: Cells treated with DMSO).

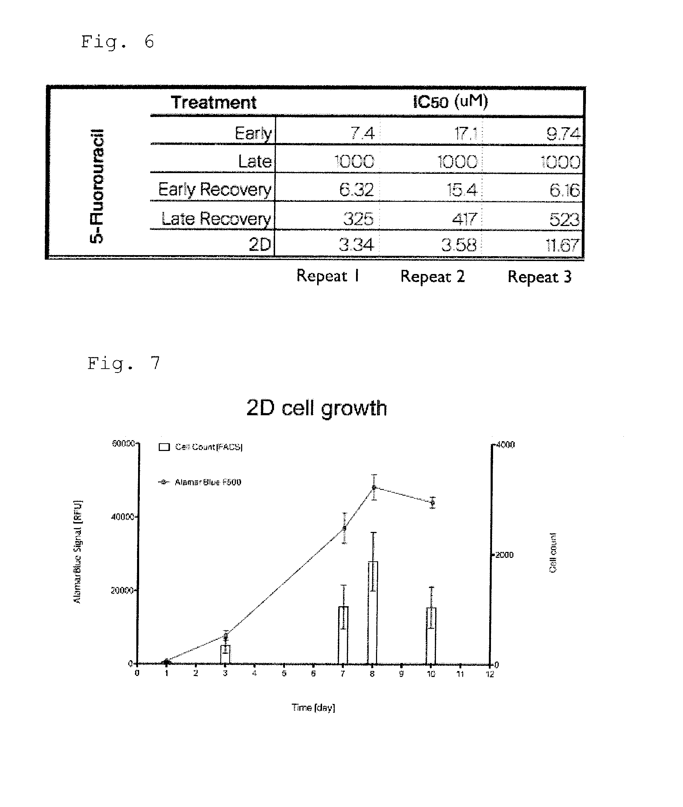

In figure 4 the determination of the half maximal inhibitory concentration (IC50) as an Alamar Blue@ readout of selected "Hits" in figure 2, example 5-Fluorouracil (5-FU), on colonies of Colon cancer HCT 116 cells is shown. The readout was performed immediately after "early-" and "late-stage" treatments of cells in a three-dimensional matrix. Dose-dependent drug efficacy is shown in figure 4 A. The arrow shows a shift to higher IC50 concentrations from "early" to "late" treatment. This indicates that 5-FU is less potent in treatment of late-stage cancers, and it confirms the "no Hit" obtained for this drug in "late-stage" drug screening as in Fig 3. The IC50 curves obtained with two-dimensional reference assays are often similar to "early-stage" treatment conditions of the present invention (three-dimensional). This is also shown in the drug screening correlation of most "Hits" observed with the two assay methods in Figure 3 A (upper panel). A summary of determined IC50 concentrations in different repeat experiments is given in figure 6. In Figure 4 B an image-based analysis of cancer cell spheroids grown in a three-dimensional matrix after "early-" and "late-stage" treatment at selected drug concentrations, IC50 and 1 mM, are shown. The upper panel shows spheroid size distribution, the lower panel the corresponding calcein-based fluorescent images. 5-FU showed a strong inhibition of spheroid growth in "early", but not in "late-stage" treatment as observed also in the metabolic readout analysis (figures 3A and 4A). These results are in line with the clinical use of 5-FU, which is no longer employed as a single agent to cure colon cancer in the clinics.

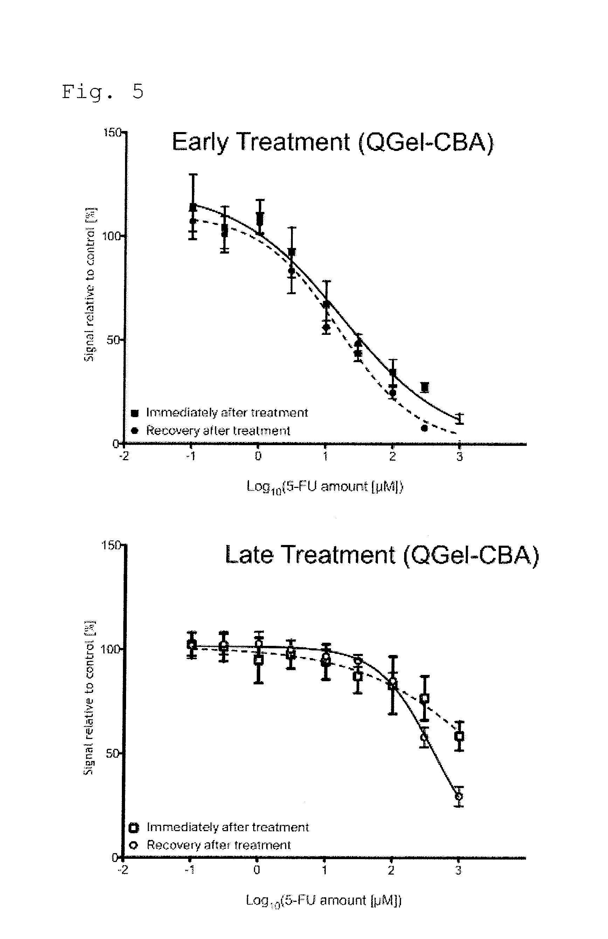

Figure 5 shows a comparison of drug efficacy immediately after the end of the treatment and after a drug-free period, named as "recovery". Here, 5-FU treatment on colonies of colon cancer cells is given as an example. Dose dependant efficacy profiles for "early-" (left panel) and "late-stage" treatment schedule (right panel) were measured before and after the drug-free recovery period of four days according to the treatment schedule described in figure 2. No significant differences were observed before and after the drug-free period in IC50 curves and concentrations (Figure 6). Interestingly, with another cancer cell derived from pancreas and the drug Gemcitabine the drug-free recovery period was a key for detection of drug efficacy using the present invention (Figure 10). The present invention allows identification of drugs even when the effect of the applied drug is detectable after a drug-free period.

Figure 6 shows a summary of IC50 concentrations of the drug 5-FU determined using the present invention and reference two-dimensional cell culture assays with colon cancer cells. The results of three independent repeat experiments are shown.

Cancer cells normally grow as two-dimensional monolayer on tissue culture plastic as described by cell suppliers and scientific publications.

In figure 7 the growth in a two-dimensional environment of colon cancer HCT 116 cells are shown. Upon reaching confluence, the whole tissue culture plastic surface is covered by cells, the cells stop growing normally due to space limitations. Disadvantageously, the in vitro situation does not appropriately reflect the physiological in vivo state (e.g. cell layer in two-dimensional methods vs. three-dimensional cell agglomeration in vivo or in three-dimensional culture methods; all cells exposed to nutrients and oxygen supply in two dimensional in vitro vs. low nutrients and oxygen conditions for cells inside spheroids in vivo or in three-dimensional culture methods).

In figure 8 cells from different cancer types display significant differences in colony growth and spheroid formation reflecting key in vivo physiological characteristics, in particular when growing from single cells in three-dimensional environments as opposed to other three-dimensional cell culture methods based on cell aggregation, in which spheroid size and growth depend on number of aggregated cells. Images of cells on the left hand side are at day fourteen. Three cancer cell lines, Colon HCT 116, Pancreatic Panc-1 and Ovarian cancer cells, were compared. Calcein-based fluorescent images of spheroids grown in three-dimensional matrix at day fourteen are shown on the left side of A). On the right, image-based analysis represented as histograms quantifying the distribution of spheroid sizes formed by cancer cells at day fourteen are shown. In general ovarian cancer cells formed largest cancer cell spheroids and Pancreas cells smallest cancer cell spheroids from the set of cancer cells tested, which is comparable to in vivo tumour growth. In B), spheroid growth curves of different cancer cells were determined using Alamar Blue@ metabolic activity fluorescence readout. Ovarian and colon cancer cells reached their growth plateau between day eleven and fifteen, pancreas cancer cells at day twenty one. Ovarian and colon cancer cell colony formation displayed faster initial growth kinetics with earlier flattening compared to pancreas cancer cells.

In figure 9 a treatment schedule within the cancer cell HCT 116 growth profile is shown for the present invention using cells embedded in a three-dimensional physiological environment (upper panel). For comparison, a two-dimensional reference assay (lower panel) is shown. The two-dimensional reference assay only covers the initial growth phase between day one and day four. Splitting of drug treatment as in the present invention covers a "three-dimensional early treatment period" (day four to seven) and a "three-dimensional late treatment period" (day fourteen to seventeen). The former is characterized by an initial growth phase of spheroids showing relatively small spheroids, no hypoxic conditions and high proliferation, and the latter by a stationary phase showing large spheroid size, hypoxic conditions and arrested cell proliferation. This is in line with in vivo tumour growth.

Figure 10 displays Pancreatic Panc-1 cancer cells treated with Gemcitabine. The dose-dependant efficacy profiles for "late-stage" treatment with Gemcitabine on pancreatic cancer cells culture in three-dimensional matrices were measured according to Alamar Blue@ readout before and after the drug-free recovery period of four to seven days. According to readouts from two-dimensional reference assays and three-dimensional assays measured immediately after the treatment, Gemcitabine was not shown to be active. However, when measurements were performed after a drug-free recovery period Gemcitabine was observed to be a potent cell growth inhibitor of cancer cells grown in a three-dimensional environment. Gemcitabine is used as a single agent in the clinics to cure pancreatic cancer. With the present invention potent drug substance are identified which are effective under certain circumstances, e.g. hypoxic conditions or stationary cell proliferation, and/or which show a delayed efficacy on cells.

## Claims

1. A method for a cell-based drug screening assay comprising the steps of:
- culturing a cell population in a culturing environment,
- defining at least two monitoring points in time in dependency of at least one of the cell types, physiological characteristics of the cells, physiological characteristics of formed-tissue, the mode of action of a drug substance and the culturing environment,
- applying drug substances to the cultured cells at least at one treatment point in time,
- monitoring of the effect of said drug substance on cells or formed tissues at least at said two monitoring points in time.

2. The method of claim 1, wherein said monitoring points in time are selected from the group consisting of
- a time point relevant for an early-stage of a specific cell, preferably between four and seven days after culturing the cells in the environment, and
- a time point for a late-stage, preferably fourteen to seventeen days after culturing the cells in the environment, and at a point in time defined by the absence or presence of a specific physiological characteristic e.g. hypoxia or change in cell growth kinetics,
and
- a time point relevant for measuring drug efficacy after a drug-free recovery phase.

3. The method of claim 1 and 2, wherein monitoring of cell proliferation continues one to seven or more days after termination of the drug application.

4. The method of claims 1 to 3, wherein cells are cultured in an environment, which comprises a structural compound and a linker compound, preferentially comprising a multi-branched polyethylene glycol with vinyl sulfone end groups and a peptide comprising at least two cysteines or, generally thiol groups enabling polymerizing to a three-dimensional matrix.

5. The method of claims 1 to 4, wherein said three-dimensional environment promotes three-dimensional growth of the encapsulated cells, preferentially spheroid growth.

6. The method of claim 1 to 5, wherein said three-dimensional environment is cast in a recipient such as a gel casting device or a standard cell-culture device, preferably a 384-well plate.

7. The method of claim 1 to 6, wherein said cells is selected of the group of any type of cell, cell line, differentiated cell, and cancer cell such as colon, pancreatic and ovarian cancer cell.

8. The method of claim 1 to 6, wherein said cells are cells of which the growth is promoted by the drug treatment.

9. The method of claim 1 to 8, wherein said drug substances are selected of the group of
(i) any chemical, natural or synthetic substance and any substance of known drug screens; or
(ii) any combination of substances according to i);
(iii) any substance(s) according to i) or ii), either in solution or combined with any carrier, nanoparticle or delivery device.

10. The method of claim 1 to 9, wherein monitoring of cell characteristics is assayed by
metabolic activity
and/or flow cytometrie,
and/or DNA content,
and/or protein quantification,
and/or cell counting,
and/or image-based analysis,
and/or levels of hypoxic and angiogenic markers,
and/or Ribonucleic acid (RNA) levels,
and/or protein expression levels,
and/or mass spectrometry,
and/or immunohistochemistry.

11. The method of claim 1 to 10, wherein the maximally tolerated dose of a drug substance is determined for a given type of cell.

12. The method of claim 1 to 11, wherein the lowest effective dose of a drug substance is determined for a given type of cell.

13. The use of the method of claim 1 to 12 for evaluating
(i) reprogramming of differentiated cells with respect to an applied substance and
(ii) differentiation of stem cells with respect to an applied substance.
